# EUROPÄISCHE PATENTSCHRIFT

(11) **EP 3 793 624 B1**
(45) Veröffentlichungstag und Bekanntmachung des Hinweises auf die Patenterteilung: **10.05.2023**
(21) Anmeldenummer: 19724470.0
(22) Anmeldetag: 14.05.2019
(51) Int. Cl.: A61L 27/46, A61L 27/58

(54) **IMPLANTAT ZUM BEHANDELN EINES KNOCHENDEFEKTS**
IMPLANT FOR TREATMENT OF A BONE DEFECT
IMPLANT DE TRAITEMENT D'UN DÉFAUT OSSEUX

(30) Priorität: 15.05.2018 DE 102018207529
(43) Veröffentlichungstag der Anmeldung: 24.03.2021
(73) Patentinhaber: Aesculap AG, 78532 Tuttlingen (DE)
(72) Erfinder: HETTICH, Georg, 79117 Freiburg (DE); ABELE, Wolfgang, 78532 Tuttlingen (DE)
(74) Vertreter: Patentanwälte Ruff, Wilhelm, Beier, Dauster & Partner mbB
(86) Internationale Anmeldenummer: PCT/EP2019/062394
(87) Internationale Veröffentlichungsnummer: WO 2019/219712

(56) Entgegenhaltungen:
- WO-A2-01/66044
- DE-A1-102012 213 246

## Beschreibung

### ANWENDUNGSGEBIET UND STAND DER TECHNIK

Die Erfindung betrifft ein Implantat, vorzugsweise zum Behandeln eines Knochendefekts.

Knochendefekte (oder sogenannte knöcherne Defekte) entstehen durch Verlust von Knochengewebe. Der Knochenverlust kann die Folge einer Knochenfraktur, eines Knochentraumas, einer Nekrose, von Osteoporose, einer Knochenerkrankung wie Tumorerkrankung, einer fehlenden mechanischen Belastung (sogenanntes Stress Shielding) oder die Folge einer chirurgischen Intervention/Reintervention, insbesondere einer Revision nach einer totalen Endoprothese, sein.

Klinisch bedeutsam sind beispielsweise azetabuläre Defekte, d.h. Defekte des Azetabulums (Hüftpfanne oder Hüftgelenkspfanne), welche insbesondere bei Revisionsoperationen am Hüftgelenk ein schwerwiegendes Problem darstellen.

Allgemein werden bei einer Revisionsoperation die drei nachfolgenden Ziele verfolgt.

Zunächst muss das ursprüngliche Gelenkzentrum wiederhergestellt werden.

Weiterhin wird eine stabile Fixierung des zum Behandeln eines Knochendefekts eingesetzten Implantats angestrebt. Insoweit wird zwischen einer sogenannten Primärstabilität und einer sogenannten Sekundärstabilität unterschieden. Unter der Primärstabilität wird die auf Reibung, Übermaß (Presssitz) oder Befestigungselementen, wie beispielsweise Knochenschrauben, beruhende Fixierung des Implantats während oder nach einer Operation, insbesondere während der ersten vier Wochen nach einer Operation, verstanden. Unter der Sekundärstabilität wird die auf knöchernen Anwachsungen beruhende Langzeit-Fixierung des Implantats, insbesondere während eines Zeitraums von mehr als einem Monat bis zu mehreren Jahren nach einer Operation, verstanden.

Schließlich wird bei einer Revisionsoperation das Ziel der sogenannten biologischen Rekonstruktion von knöchernen Defekten angestrebt. Unter der biologischen Rekonstruktion eines Knochendefekts werden allgemein die Durchdringung und/oder der Wiederaufbau des Knochendefekts mit patienteneigenem Knochengewebe und/oder natürlichen Knochenersatzmaterialien und/oder künstlichen Knochenersatzmaterialien verstanden.

Bei Knochendefekten wird allgemein zwischen umschlossenen Knochendefekten (sogenannte "contained"-Defekte) und nicht umschlossenen Knochendefekten (sogenannte "uncontained"-Defekte) unterschieden. Umschlossene Knochendefekte liegen vor, wenn vitaler Patientenknochen und ein eingebrachtes Implantat den Knochendefekt vollständig umschließen. Dagegen werden Knochendefekte als nicht umschlossene Defekte klassifiziert, wenn ein eingebrachtes Implantat den Knochendefekt nicht vollständig umschließt, d.h. offene Stellen zurückbleiben, welche nicht vom Implantat oder Patientenknochen überdeckt sind.

Für die Behandlung umschlossener Knochendefekte hat sich in der Praxis die sogenannte "Impaction Bone Grafting"-Technik durchgesetzt. Bei dieser Technik werden aus einem allogenen Knochenmaterial, wie beispielsweise einem Femurkopf, Knochenchips hergestellt und mittels eines geeigneten Instruments, in der Regel mittels eines sogenannten Nachschlägers, in einem Knochendefekt verdichtet. Bei dieser Technik können grundsätzlich alle drei vorgenannten Ziele der Revisionsoperation erreicht werden. Allerdings bergen allogene Knochenchips trotz Thermobehandlung ein potenzielles Infektionsrisiko. Auch ist die intraoperative Handhabung von Knochenchips kompliziert und zeitintensiv. Darüber hinaus sind die Kosten für allogene Knochenchips aufgrund gestiegener regulatorischer Anforderungen nicht unerheblich.

Ein weiterer Ansatz zur Behandlung von Knochendefekten besteht in der Verwendung einer sogenannten demineralisierten Knochenmatrix (demineralized bone matrix, DBM). Eine derartige Knochenmatrix ist durch Entfernung des mineralischen Knochenanteils aus Knochen erhältlich. Nachteilig hierbei ist die fehlende mechanische Festigkeit. Zudem existiert auch in diesem Fall ein potenzielles Infektionsrisiko, da demineralisierte Knochenmaterialien üblicherweise aus tierischem oder menschlichem Knochen gewonnen werden.

Ein weiterer Ansatz zur Behandlung von Knochendefekten betrifft die Verwendung von granulatförmigen Calciumphosphaten. Calciumphosphat ähnelt grundsätzlich dem mineralischen Anteil von Knochen und hat sich als besonders biokompatibel erwiesen. Nachteilig bei der Verwendung von Calciumphosphaten ist jedoch ebenfalls eine geringe mechanische Festigkeit.

Ein weiterer Ansatz zur Behandlung von Knochendefekten besteht schließlich in der Verwendung von metallischen Gitterstrukturen. Diese sind jedoch mit dem Nachteil behaftet, dass eine biologische Rekonstruktion des Knochendefekts in der Regel nicht stattfindet. Weiterhin nachteilig ist, dass im Falle einer Revision die metallischen Gitterstrukturen in der Regel ausgetauscht werden müssen, was zu einer Defektvergrößerung führt.

Oligopodenförmige Stützkörper zur Behandlung von Knochendefekten sind aus der DE 10 2012 213 246 A1 bekannt.

Ein Knochenersatzmaterial mit Stützkörpern und einer modellierfähigen sowie bei Kontakt mit Wasser oder einer wässrigen Flüssigkeit aushärtbaren Masse ist Gegenstand der DE 10 2015 209 007 A1.

Ein Implantat zum Behandeln von Knochendefekten, welches eine poröse Gerüststruktur aus miteinander fusionierten, größtenteils beschichteten Granulatpartikeln sowie eine die Gerüststruktur abschnittsweise umgebende Barrieremembran enthält, ist aus der US 7,731,756 B2 bekannt.

Eine Vorstufe für die Herstellung eines pastösen Knochenersatzmaterials ist in der WO 2008/077257 A1 beschrieben. Die Vorstufe umfasst eine biokompatible Substanz, welche durch Einwirkung von Wasser oder einer wässrigen Lösung zu einem Hydrogel quellbar ist, sowie Feststoffpartikel, welche aus einer als Knochenersatzmaterial tauglichen Substanz bestehen.

Gegenstand der WO 2008/028466 A2 ist eine plastisch verformbare Zubereitung zum Knochenaufbau/-ersatz. Die Zubereitung basiert auf Keramik-Suspensionen, welche eine partikuläre, poröse, in Wasser gering lösliche Komponente und mindestens eine in Wasser oder Alkohol gelöste, flüssige, wachsartige oder gelartige Komponente aufweisen, so dass ein pastöses Gemisch ausgebildet ist.

Aus der WO 01/66044 A2 ist ein suspensionsmaterialhaltiges Knochenersatzmaterial bekannt, das keramische Partikel sowie polymere Partikel aufweist.

### AUFGABE UND LÖSUNG

Aufgabe der Erfindung ist es, ein Implantat bereitzustellen, welches sich vorzugsweise zum Behandeln eines Knochendefekts, insbesondere eines periprothetischen Knochendefekts, eignet und die einleitend genannten Nachteile, insbesondere im Zusammenhang einer Hüftgelenksrevision oder anderen Gelenksrevisionen, teilweise oder vollständig vermeidet. Das Implantat soll insbesondere allen drei im Zusammenhang einer Revisionsoperation vorgenannten Zielen, nämlich der Wiederherstellung des Gelenkzentrums, der Gewährleistung einer ausreichenden Primärstabilität und Sekundärstabilität des Implantats sowie der biologischen Rekonstruktion des Knochendefekts, gerecht werden.

Weiterhin stellt sich die Erfindung die Aufgabe, ein Herstellungsverfahren für ein derartiges Implantat, einen Kit sowie ein Verfahren zum Behandeln eines Knochendefekts bereitzustellen. Hinsichtlich des Implantats wird die der Erfindung zugrunde liegende Aufgabe durch ein Implantat mit den Merkmalen gemäß unabhängigem Anspruch 1 gelöst. Bevorzugte Ausgestaltungen des Implantats können den jeweiligen Unteransprüchen sowie der Beschreibung entnommen werden.

Die Erfindung betrifft ein Implantat, vorzugsweise zum Behandeln, insbesondere Auskleiden und/oder Abdichten und/oder Unterfüttern und/oder wenigstens teilweisen Auffüllen und/oder biologischen Rekonstruieren, eines Knochendefekts.

Bei dem Implantat handelt es sich vorzugsweise um ein chirurgisches Implantat, welches im Sinne der vorliegenden Erfindung auch als Knochenersatzmaterial bezeichnet werden kann. Das Implantat weist Folgendes auf oder besteht aus Folgendem:
- eine Bindemittelmatrix,
- eine erste Gruppe von Stützkörpern und
- eine zweite Gruppe von Stützkörpern.

Die Stützkörper der ersten Gruppe und die Stützkörper der zweiten Gruppe sind von der Bindemittelmatrix wenigsten teilweise, insbesondere nur teilweise oder vollständig, umgeben.

Bevorzugt sind die Stützkörper der ersten Gruppe und die Stützkörper der zweiten Gruppe innerhalb der Bindemittelmatrix angeordnet.

Weiterhin bevorzugt sind die Stützkörper der ersten Gruppe und die Stützkörper der zweiten Gruppe relativ zueinander beweglich.

Besonders bevorzugt sind die Stützkörper der ersten Gruppe und die Stützkörper der zweiten Gruppe jeweils als einzelne oder lose, d.h. nicht miteinander verbundene, Stützkörper gestaltet. Dadurch ist jeder einzelne Stützkörper unabhängig von den anderen Stützkörpern, vorzugsweise innerhalb der Bindemittelmatrix, beweglich, wodurch eine Anpassung des Implantats an patientenindividuelle Knochendefektformen erleichtert wird.

Weiterhin ist es bevorzugt, wenn sich die Stützkörper der ersten Gruppe und/oder die Stützkörper der zweiten Gruppe, insbesondere ein Teil davon, gegenseitig berühren. Besonders bevorzugt berühren sich die Stützkörper der ersten Gruppe und die Stützkörper der zweiten Gruppe, insbesondere ein Teil davon, gegenseitig.

Insbesondere können sich die Stützkörper der ersten Gruppe und/oder die Stützkörper der zweiten Gruppe, insbesondere ein Teil davon, in einem impaktierten, d.h. verdichteten und/oder gepressten, Zustand des Implantats gegenseitig berühren. Bevorzugt berühren sich die Stützkörper der ersten Gruppe und die Stützkörper der zweiten Gruppe, insbesondere wenigstens ein Teil davon, in einem impaktierten, d.h. verdichteten und/oder gepressten, Zustand des Implantats gegenseitig.

Das Implantat gemäß der vorliegenden Erfindung zeichnet sich besonders dadurch aus, dass
- die Bindemittelmatrix mittels Wasser und/oder einer wässrigen Flüssigkeit entfernbar ist und die Stützkörper der ersten Gruppe schneller in vivo abbaubar und/oder schneller in vivo resorbierbar sind als die Stützkörper der zweiten Gruppe
   und/oder
- die Bindemittelmatrix schneller in vivo abbaubar und/oder schneller in vivo resorbierbar ist als die Stützkörper der ersten und die Stützkörper der zweiten Gruppe und die Stützkörper der ersten Gruppe schneller in vivo abbaubar und/oder schneller in vivo resorbierbar sind als die Stützkörper der zweiten Gruppe.

Der Ausdruck "Knochendefekt" bedeutet im Sinne der vorliegenden Erfindung einen durch Verlust von Knochengewebe, insbesondere Gelenksknochengewebe, vorzugsweise Hüftgelenks- oder Kniegelenksknochengewebe, oder von Wirbelkörpergewebe betroffenen Knochenbereich, insbesondere Gelenksknochenbereich, vorzugsweise Hüftgelenks- oder Kniegelenksknochenbereich, oder Wirbelkörperbereich. Der Knochenverlust kann, wie einleitend bereits erwähnt, die Folge einer Knochenfraktur, eines Knochentraumas, einer Knochenerkrankung wie Tumorerkrankung, von Osteoporose oder einer chirurgischen Intervention oder Reintervention, insbesondere einer Revision nach einer totalen Hüft- oder Kniearthroplastik, sein.

Insbesondere kann der Ausdruck "Knochendefekt" im Sinne der vorliegenden Erfindung einen periprothetischen Knochendefekt, d.h. einen durch periprothetischen Knochengewebsverlust, insbesondere aufgrund von mechanischer Überlastung und/oder Abrieb-induzierter Osteolyse und/oder Implantatmigration, betroffenen Knochenbereich bedeuten.

Vorzugsweise handelt es sich bei dem Knochendefekt um einen Gelenksknochendefekt, insbesondere Kniegelenksknochendefekt oder Hüftgelenksknochendefekt, bevorzugt Azetabulumdefekt.

Bei dem Knochendefekt im Sinne der vorliegenden Erfindung kann es sich weiterhin um einen menschlichen Knochendefekt oder einen tierischen Knochendefekt handeln.

Unter dem Ausdruck "tierischer Knochendefekt" soll im Sinne der vorliegenden Erfindung ein Knochendefekt eines nicht humanen Säugetiers, wie beispielsweise eines Pferds, einer Kuh, einer Ziege, eines Schafs, eines Schweins oder eines Nagetiers, wie beispielsweise eines Hasen, einer Ratte oder einer Maus, verstanden werden.

Unter dem Ausdruck "Stützkörper" sollen im Sinne der vorliegenden Erfindung Körper, insbesondere partikuläre Körper, verstanden werden, welche dazu ausgebildet sind, in einem zu behandelnden Knochendefekt üblicherweise auftretenden Kräften ohne Deformation oder Zerstörung, wenigstens jedoch ohne wesentliche Deformation bzw. Zerstörung, zu widerstehen und mithin lasttragende Funktionen zu übernehmen. Die Stützkörper können daher im Sinne der vorliegenden Erfindung auch als lasttragende Stützkörper bezeichnet werden.

Unter dem Ausdruck "Bindemittelmatrix" soll im Sinne der vorliegenden Erfindung eine Matrix oder Struktur verstanden werden, welche dazu ausgebildet ist, die Stützkörper der ersten Gruppe sowie die Stützkörpern der zweiten Gruppe zusammenzuhalten, ohne dass eine unkontrollierte Dislokation der Stützkörper beim Einbringen des Implantats in einen zu behandelnden Knochendefekt stattfindet.

Unter dem Ausdruck "in vivo abbaubar" soll im Sinne der vorliegenden Erfindung ein Stoff oder Material verstanden werden, welcher bzw. welches in einem menschlichen oder tierischen Körper, insbesondere unter der Einwirkung von Enzymen, metabolisiert werden kann. Der Abbau des Stoffs bzw. Materials kann vollständig bis zur Mineralisierung, d.h. Freisetzung von chemischen Elementen und deren Einbau in anorganische Verbindungen, wie beispielsweise Kohlendioxid und/oder Sauerstoff und/oder Ammoniak, verlaufen, oder auf der Stufe von abbaustabilen Zwischen- oder Transformationsprodukten stehen bleiben.

Unter dem Ausdruck "in vivo resorbierbar" soll im Sinne der vorliegenden Erfindung ein Stoff oder ein Material verstanden werden, welcher bzw. welches in einem menschlichen oder tierischen Körper durch lebende Zellen oder lebendes Gewebe, wie beispielsweise Nieren, aufgenommen werden kann, ohne dass ein Abbau oder ohne dass ein nennenswerter Abbau des Stoffs bzw. Materials stattfindet.

Unter dem Ausdruck "wässrige Flüssigkeit" soll im Sinne der vorliegenden Erfindung eine wasserhaltige, d.h. eine Wasser enthaltende, Flüssigkeit verstanden werden. Bei der wässrigen Flüssigkeit kann es sich insbesondere um eine medizinisch verträgliche, wässrige Spülflüssigkeit, bevorzugt um eine medizinisch verträgliche, wässrige Spüllösung, handeln.

Mittels des erfindungsgemäßen Implantats lassen sich insbesondere die nachfolgenden Vorteile realisieren:
- Mittels des erfindungsgemäßen Implantats lassen sich alle drei einleitend genannten Ziele einer Revisionsoperation an einem Gelenk, insbesondere an einem Hüftgelenk, realisieren.
- Im Falle einer mittels Wasser und/oder einer wässrigen Flüssigkeit entfernbaren Bindemittelmatrix kann diese durch den Chirurgen noch während einer intra-operativen Phase aus dem zu behandelnden Knochendefekt entfernt, insbesondere herausgespült, werden. Dadurch können rasch Räume oder Plätze für einwachsendes Knochengewebe geschaffen und mithin die biologische Rekonstruktion des Knochendefekts initiiert werden. Die Primärstabilität des Implantats wird während dieser Phase durch die Stützkörper der ersten Gruppe sowie durch die Stützkörper der zweiten Gruppe gewährleistet.
   Im Falle einer im Verhältnis zu den Stützkörpern der ersten und zweiten Gruppe schneller in vivo abbaubaren und/oder schneller in vivo resorbierbaren Bindemittelmatrix können während einer ersten Heilungsphase die für ein anfängliches Einwachsen von Knochengewebe erforderlichen Räume bzw. Plätze aufgrund eines nach erfolgter Implantation stattfindenden in vivo Abbaus und/oder aufgrund einer nach erfolgter Implantation stattfindenden in vivo Resorption der Bindemittelmatrix bereitgestellt werden. Auch in diesem Fall wird die Primärstabilität des Implantats durch die Stützkörper der ersten Gruppe sowie durch die Stützkörper der zweiten Gruppe gewährleistet.
- Während einer zweiten Heilungsphase werden dann als nächstes, wenigstens hauptsächlich, die Stützkörper der ersten Gruppe in vivo abgebaut und/oder in vivo resorbiert. Der in vivo Abbau und/oder die in vivo Resorption der Stützkörper der ersten Gruppe geht mit deren Ab- und/oder Umbau zu Knochen einher, so dass die biologische Rekonstruktion des Knochendefekts kontinuierlich voranschreiten kann. Während dieser Phase wird die Sekundärstabilität des Implantats durch die Stützkörper der zweiten Gruppe gewährleistet.
- Während einer dritten Heilungsphase werden schließlich die Stützkörper der zweiten Gruppe in vivo abgebaut und/oder in vivo resorbiert, wodurch diese ebenfalls zu Knochen ab- und/oder umgebaut werden, so dass auch während dieser Phase eine kontinuierliche biologische Rekonstruktion des Knochendefekts gewährleistet ist. Zwar nimmt die auf die Stützkörper der zweiten Gruppe zurückgehende mechanische Stabilisierung des Implantats mit deren Abbau und/oder Resorption zunehmend ab. Dies wird jedoch durch die zunehmend voranschreitende biologische Rekonstruktion des Knochendefekts mehr als aufgefangen. Nach vollständigem in vivo Abbau und/oder vollständiger in vivo Resorption der Stützkörper der zweiten Gruppe ist die dritten Phase und mithin die biologische Rekonstruktion des Knochendefekts abgeschlossen, wobei die mechanische Stabilität über den gesamten Zeitraum der biologischen Rekonstruktion des Knochendefekts gewährleistet war.
- Mittels des erfindungsgemäßen Implantats lassen sich somit alle drei für eine erfolgreiche Revisionsoperation an einem Gelenk, insbesondere Hüftgelenk, maßgeblichen Ziele, nämlich die Wiederherstellung des Rotationszentrums, die Gewährleistung einer ausreichenden Primärstabilität und Sekundärstabilität des Implantats sowie die biologische Rekonstruktion des Knochendefekts, realisieren.

In Ausgestaltung der Erfindung ist die Bindemittelmatrix plastisch verformbar, insbesondere knetförmig oder knetbar, gestaltet. Eine derartig gestaltete Bindemittelmatrix lässt sich problemlos intra-operativ an die Form des zu behandelnden Knochendefekts anpassen. Insbesondere kann eine plastisch verformbare Bindemittelmatrix in den Knochendefekt gedrückt oder gepresst werden, bis sich die darin befindlichen Stützkörper zumindest teilweise gegenseitig berühren und eine weitere Verdichtung des Implantats verhindern. Insgesamt verbessert eine plastisch verformbare Bindemittelmatrix daher mit besonderem Vorteil die intraoperative Handhabung des Implantats.

In weiterer Ausgestaltung der Erfindung handelt es sich bei der Bindemittelmatrix um eine wasserlösliche Bindemittelmatrix. Eine in Wasser lösliche Bindemittelmatrix hat den Vorteil, dass sie sich besonders rasch intra-operativ entfernen lässt.

In weiterer Ausgestaltung der Erfindung weist die Bindemittelmatrix ein anderes Material auf oder besteht aus einem anderen Material als die Stützkörper der ersten Gruppe und die Stützkörper der zweiten Gruppe.

In weiterer Ausgestaltung der Erfindung weist die Bindemittelmatrix Polyethylenglykol auf oder besteht aus Polyethylenglykol. Eine solche Bindemittelmatrix hat generell den Vorteil, dass sie kein potenzielles Infektionsrisiko für den Patienten beinhaltet. Des Weiteren hat Polyethylenglykol den Vorteil, dass es von den meisten Körperflüssigkeiten schnell ausgespült und im Normalfall ohne Komplikationen über die Niere ausgeschieden wird.

Vorzugsweise ist das Polyethylenglykol ausgewählt aus der Gruppe bestehend aus PEG 400, PEG 1500 und Mischungen davon.

Unter dem Ausdruck "Polyethylenglykol 400" soll im Sinne der vorliegenden Erfindung ein Polyethylenglykol mit einer durchschnittlichen Anzahl von 400 (gesprochen: vierhundert) Struktureinheiten -CH₂-CH₂-O- pro Molekül verstanden werden.

Unter dem Ausdruck "Polyethylenglykol 1.500" soll im Sinne der vorliegenden Erfindung ein Polyethylenglykol mit einer durchschnittlichen Anzahl von 1.500 (gesprochen: eintausendfünfhundert) Struktureinheiten -CH₂-CH₂-O- pro Molekül verstanden werden.

In weiterer Ausgestaltung der Erfindung weist die Bindemittelmatrix eine Mischung von PEG 400 und PEG 1500 auf oder besteht aus einer solchen Mischung. Die genannte Mischung hat den Vorteil, dass sie die erforderliche Festigkeit aufweist, um die Stützkörper zu binden und dadurch die Handhabungseigenschaften für den Chirgurgen zu verbessern.

In weiterer Ausgestaltung der Erfindung weist die Bindemittelmatrix ein Verhältnis von PEG 400 zu PEG 1500 von 1,5 : 1 bis 4 : 1 auf. Dieses Mischungsverhältnis hat sich als besonders vorteilhaft im Hinblick auf Festigkeit und Handhabung herausgestellt.

In weiterer Ausgestaltung der Erfindung weist die Bindemittelmatrix ferner Glycerin (Propan-1,2,3-triol) auf. Durch die Zugabe von Glycerin wird mit besonderem Vorteil die Auflösung und/oder der Ausspülprozess der Bindemittelmatrix unterstützt.

Das Glycerin kann einen Anteil von 0,1 Gew.-% bis 30 Gew.-%, insbesondere 0,5 Gew.-% bis 20 Gew.-%, bevorzugt 1 Gew.-% bis 10 Gew.-%, aufweisen, bezogen auf das Gesamtgewicht des Implantats.

In weiterer Ausgestaltung der Erfindung weist die Bindemittelmatrix ein Verhältnis von PEG 400 zu PEG 1500 zu Glycerin von 3 : 2 : 1 oder 4 : 1 : 1 auf. Die Zugabe von Glycerin in diesem Verhältnis führt zu besonders guten Handlingseigenschaften in Kombination mit einer schnellen Auflösung in-vivo.

Ferner kann es bevorzugt sein, dass die Bindemittelmatrix Kollagen aufweist oder aus Kollagen besteht. Insbesondere kann die Bindemittelmatrix eine Kollagenmatrix aufweisen oder aus einer Kollagenmatrix bestehen.

Bei dem Kollagen kann es sich um humanes Kollagen oder xenogenes, d.h. nicht humanes, Kollagen, handeln. Beispielsweise kann das Kollagen bovinen, porcinen oder equinen Ursprungs sein.

Grundsätzlich kann das Kollagen ausgewählt sein aus der Gruppe bestehend aus Kollagen Typ I, Kollagen Typ II, Kollagen Typ III, Kollagen Typ IV, Kollagen Typ V, Kollagen Typ VI, Kollagen Typ VII, Kollagen Typ VIII, Kollagen Typ VIIII, Kollagen Typ X, Kollagen Typ XI, Kollagen Typ XII, Kollagen Typ XIII, Kollagen Typ XIIII, Kollagen Typ XV, Kollagen Typ XVI, Kollagen Typ XVII, Kollagen Typ XVIII, Kollagen Typ XVIIII, Kollagen Typ XX, Kollagen Typ XXI, Kollagen Typ XXII, Kollagen Typ XXIII, Kollagen Typ XXIIII, Kollagen Typ XXV, Kollagen Typ XXVI, Kollagen Typ XXVII, Kollagen Typ XXVIII und Mischungen von wenigstens zwei der genannten Kollagentypen.

Bevorzugt ist das Kollagen ausgewählt aus der Gruppe bestehend aus Kollagen Typ I, Kollagen Typ II, Kollagen Typ III, Kollagen Typ IV, Kollagen Typ V und Mischungen von wenigstens zwei der genannten Kollagentypen.

Eine Bindemittelmatrix aufweisend oder bestehend aus Kollagen hat den Vorteil einer osteostimulativen Wirkung. Dadurch kann Knochenwachstum insbesondere in Zwischenräumen der Bindemittelmatrix angeregt werden.

In weiterer Ausgestaltung der Erfindung weist die Bindemittelmatrix einen Anteil von 10 Gew.-% bis 60 Gew.-%, insbesondere 20 Gew.-% bis 50 Gew.-%, bevorzugt 35 Gew.-% bis 45 Gew.-%, auf, bezogen auf das Gesamtgewicht des Implantats. Insbesondere durch einen derartigen Bindemittelmatrixanteil des Implantats kann sichergestellt werden, dass während einer anfänglichen Heilungsphase des Knochendefekts ausreichend Platz oder Raum für das Einwachsen von Knochengewebe geschaffen wird.

In weiterer Ausgestaltung der Erfindung weisen die Stützkörper der ersten Gruppe ein anderes Material auf oder bestehen aus einem anderen Material als die Stützkörper der zweiten Gruppe.

In weiterer Ausgestaltung der Erfindung weisen die Stützkörper der ersten Gruppe β-Tricalciumphosphat auf. Alternativ können die Stützkörper der ersten Gruppe aus β-Tricalciumphosphat bestehen.

β-Tricalciumphosphat hat den Vorteil, dass es eine schnellere in-vivo-Abbaugeschwindigkeit und/oder schnellere in-vivo-Resorptionsgeschwindigkeit aufweist als andere Materialien, wie beispielsweise Hydroxylapatit oder biphasisches Calciumphosphat. Dadurch sind Stützkörper, welche β-Tricalciumphosphat aufweisen oder aus β-Tricalciumphosphat bestehen, in besonderer Weise dazu geeignet, die für eine erfolgreiche Behandlung des Knochendefekts erforderliche Primärstabilität des Implantats zu gewährleisten.

β-Tricalciumphosphat wird allgemein auch als das "echte Calciumorthophosphat" mit der stöchiometrischen Zusammensetzung Ca₃(PO4)₂ bezeichnet. Es kann nicht aus wässriger Lösung gefällt, sondern nur durch Calcinieren oberhalb 800 °C hergestellt werden, beispielsweise aus Calcium-defizitärem Hydroxylapatit gemäß nachfolgender Gleichung:

Ca₉(HPO₄)(PO₄)₅OH → 3 Ca₃(PO4)₂ + H₂O

In weiterer Ausgestaltung der Erfindung weisen die Stützkörper der zweiten Gruppe Hydroxylapatit auf. Alternativ können die Stützkörper der zweiten Gruppe aus Hydroxylapatit bestehen.

Unter Hydroxylapatit versteht man ein Calciumorthophosphat mit der stöchiometrischen Zusammensetzung Ca₁₀(PO₄)₆(OH)₂.

Die Verwendung von Hydroxylapatit für die Stützkörper der zweiten Gruppe hat den Vorteil, dass es eine langsamere in-vivo-Abbaugeschwindigkeit und/oder langsamere in-vivo-Resorptionsgeschwindigkeit besitzt als andere Materialien, wie beispielsweise β-Tricalciumphosphat. Dadurch sind Stützkörper, welche Hydroxylapatit aufweisen oder aus Hydroxylapatit bestehen, in besonderer Weise dazu geeignet, die für eine erfolgreiche Behandlung des Knochendefekts erforderliche Sekundärstabilität des Implantats zu übernehmen.

In weiterer Ausgestaltung der Erfindung weisen die Stützkörper der zweiten Gruppe biphasisches Calciumphosphat, d.h. eine Mischung von β-Tricalciumphosphat und Hydroxylapatit, auf. Alternativ können die Stützkörper der zweiten Gruppe aus biphasischem Calciumphosphat, d.h. einer Mischung von β-Tricalciumphosphat und Hydroxylapatit, bestehen.

Die im vorherigen Absatz erwähnte Mischung von β-Tricalciumphosphat und Hydroxylapatit weist bevorzugt einen Anteil an β-Tricalciumphosphat von 50 Gew.-% und einen Anteil an Hydroxylapatit von 50 Gew.-%, jeweils bezogen auf das Gesamtgewicht der Mischung, auf.

Die Verwendung von biphasischem Calciumphosphat für die Stützkörper der zweiten Gruppe hat den Vorteil, dass (auch) biphasisches Calciumphosphat aufgrund seiner langsameren in-vivo-Abbaugeschwindigkeit und/oder langsameren in-vivo-Resorptionsgeschwindigkeit gegenüber anderen Materialien, wie beispielsweise β-Tricalciumphosphat, in der Lage ist, die für eine erfolgreiche Behandlung des Knochendefekts erforderliche Sekundärstabilität des Implantats zu übernehmen.

In weiterer Ausgestaltung der Erfindung weisen die Stützkörper insgesamt, d.h. die Stützkörper der ersten Gruppe und die Stützkörper der zweiten Gruppe, einen Anteil von 40 Gew.-% bis 90 Gew.-%, insbesondere 50 Gew.-% bis 80 Gew.-%, bevorzugt 55 Gew.-% bis 65 Gew.-%, auf, bezogen auf das Gesamtgewicht des Implantats. Insbesondere durch die in diesem Absatz beschriebenen Stützkörperanteile ist eine für die Behandlung des Knochendefekts vorteilhafte Formstabilität, Primärstabilität sowie Sekundärstabilität des Implantats erzielbar.

Ferner kann es bevorzugt sein, dass die Stützkörper der ersten Gruppe oder die Stützkörper der zweiten Gruppe Bioglas aufweisen oder aus Bioglas bestehen.

Bioglas hat den Vorteil einer antibakteriellen Wirkung. Beim Abbau von Bioglas tritt eine Verschiebung des pH-Werts auf, welche eine Bakterienansiedlung auf der Oberfläche des Implantats verhindert.

Unter dem Ausdruck "Bioglas" soll im Sinne der vorliegenden Erfindung ein Material, insbesondere ein Glas oder eine Keramik, verstanden werden, welches SiO₂, P₂O₅, CaO und Na₂O aufweist oder aus diesen Bestandteilen besteht.

Beispielsweise kann es sich bei dem Bioglas um ein Bioglas mit der Bezeichnung "Bioglass 45S5" handeln. Hierbei handelt es sich um ein Bioglas, welches aus 45 Gew.-% SiO₂, 6 Gew.-% P₂O₅, 24,5 Gew.-% CaO und 24,5 Gew.-% Na₂O besteht.

Alternativ kann es sich bei dem Bioglas um ein Bioglas mit der Bezeichnung "Bioglass 42S5.6" handeln. Hierbei handelt es sich um ein Bioglas, welches aus 42,1 Gew.-% SiO₂, 2,6 Gew.-% P₂O₅, 29 Gew.-% CaO und 26,3 Gew.-% Na₂O besteht.

Alternativ kann es sich bei dem Bioglas um ein Bioglas mit der Bezeichnung "Bioglass 46S5.2" handeln. Hierbei handelt es sich um ein Bioglas, welches aus 46,1 Gew.-% SiO₂, 2,6 Gew.-% P₂O₅, 26,9 Gew.-% CaO und 24,4 Gew.-% Na₂O besteht.

Alternativ kann es sich bei dem Bioglas um ein Bioglas mit der Bezeichnung "Bioglass 52S4.6" handeln. Hierbei handelt es sich um ein Bioglas, welches aus 52,1 Gew.-% SiO₂, 2,6 Gew.-% P₂O₅, 23,8 Gew.-% CaO und 21,5 Gew.-% Na₂O besteht.

Alternativ kann es sich bei dem Bioglas um ein Bioglas mit der Bezeichnung "Bioglass 55S4.3" handeln. Hierbei handelt es sich um ein Bioglas, welches aus 55,1 Gew.-% SiO₂, 2,6 Gew.-% P₂O₅, 22,2 Gew.-% CaO und 20,1 Gew.-% Na₂O besteht.

Alternativ kann es sich bei dem Bioglas um ein Bioglas mit der Bezeichnung "Bioglass 60S3.8" handeln. Hierbei handelt es sich um ein Bioglas, welches aus 60,1 Gew.-% SiO₂, 2,6 Gew.-% P₂O₅, 19,6 Gew.-% CaO und 17,7 Gew.-% Na₂O besteht.

Alternativ kann es sich bei dem Bioglas um ein Bioglas mit der Bezeichnung "Bioglass 45S5F" handeln. Hierbei handelt es sich um ein Bioglas, welches aus 45 Gew.-% SiO₂, 6 Gew.-% P₂O₅, 12,25 Gew.-% CaO, 12,25 Gew.-% CaF₂ und 24,5 Gew.-% Na₂O besteht.

Alternativ kann es sich bei dem Bioglas um ein Bioglas mit der Bezeichnung "Bioglass 45S5.4F" handeln. Hierbei handelt es sich um ein Bioglas, welches aus 45 Gew.-% SiO₂, 6 Gew.-% P₂O₅, 14,7 Gew.-% CaO, 9,8 Gew.-% CaF₂ und 24,5 Gew.-% Na₂O besteht.

Alternativ kann es sich bei dem Bioglas um ein Bioglas mit der Bezeichnung "Bioglass 40S5B5" handeln. Hierbei handelt es sich um ein Bioglas, welches aus 40 Gew.-% SiO₂, 6 Gew.-% P₂O₅, 24,5 Gew.-% CaO, 24,5 Gew.-% Na₂O und 5 Gew.-% B₂O₃ besteht.

Alternativ kann es sich bei dem Bioglas um ein Bioglas mit der Bezeichnung "Bioglass 52S4.6" handeln. Hierbei handelt es sich um ein Bioglas, welches aus 52 Gew.-% SiO₂, 6 Gew.-% P₂O₅, 21 Gew.-% CaO und 21 Gew.-% Na₂O besteht.

Alternativ kann es sich bei dem Bioglas um ein Bioglas mit der Bezeichnung "Bioglas 55S4.3" handeln. Hierbei handelt es sich um ein Bioglas, welches aus 55 Gew.-% SiO₂, 6 Gew.-% P₂O₅, 19,5 Gew.-% CaO und 19,5 Gew.-% Na₂O besteht.

Bevorzugt sind die Stützkörper der ersten Gruppe und/oder die Stützkörper der zweiten Gruppe, insbesondere die Stützkörper der ersten Gruppe und die Stützkörper der zweiten Gruppe, derart gestaltet, dass sie, insbesondere durch Impaktieren, d.h. Verdichten und/oder Pressen, des Implantats, beispielsweise mittels eines Nachschlägers, in eine osteokonduktive Anordnung oder osteokonduktive Gerüststruktur, d.h. in eine Anordnung bzw. Gerüststruktur mit Zwischenräumen, welche ein Einwachsen von Knochengewebe erlauben, überführbar sind.

Insbesondere kann das Implantat, insbesondere in einem impaktierten, d.h. verdichteten und/oder gepressten, Zustand, eine osteokonduktive Anordnung oder osteokonduktive Gerüststruktur, d.h. eine Anordnung bzw. Gerüststruktur mit Zwischenräumen, welche ein Einwachsen von Knochengewebe erlauben, aufweisen. Die osteokonduktive Anordnung bzw. osteokonduktive Gerüststruktur beruht vorzugsweise auf einer gegenseitigen Berührung der Stützkörper. Die Zwischenräume der osteokonduktiven Anordnung bzw. Gerüststruktur sind vorzugsweise wenigstens teilweise, insbesondere nur teilweise oder vollständig, mit der Bindemittelmatrix befüllt.

Weiterhin kann die Bindemittelmatrix nur in den Zwischenräumen der osteokonduktiven Anordnung bzw. Gerüststruktur enthalten sein.

Die osteokonduktive Anordnung bzw. Gerüststruktur kann mit besonderem Vorteil eine Druckstabilität von bis zu 10 MPa aufweisen. Mit anderen Worten können Zwischenräume der osteokonduktiven Anordnung bzw. Gerüststruktur bis zu einem Druck von 10 MPa erhalten bleiben.

Weiterhin ist es bevorzugt, wenn die osteokonduktive Anordnung bzw. Gerüststruktur eine Porosität, d.h. einen Anteil an Stützkörpern (Summe aus Stützkörpern der ersten Gruppe und Stützkörpern der zweiten Gruppe) zu einem Anteil an Zwischenräumen, von 20 % bis 60 % aufweist.

Weiterhin ist es bevorzugt, wenn die Zwischenräume der osteokonduktiven Anordnung bzw. Gerüststruktur einen (absoluten) Durchmesser von 20 µm bis 1500 µm aufweisen. Unter dem Ausdruck "Durchmesser" soll an dieser Stelle der größtmögliche Abstand verstanden werden, welchen zwei gegenüberliegende Punkte zueinander entlang einer Umrandungslinie eines Zwischenraums einnehmen können.

Weiterhin ist es bevorzugt, wenn die Zwischenräume der osteokonduktiven Anordnung bzw. Gerüststruktur einen mittleren Durchmesser (bestimmt vorzugsweise nach dem Durchmesser der größten Kugel, welche komplett in einen Zwischenraum der osteokonduktiven Anordnung bzw. Gerüststruktur passt) von 300 µm bis 1000 µm aufweisen.

Weiterhin kann die osteokonduktive Anordnung bzw. Gerüststruktur eine Interkonnektivität, d.h. einen Anteil an miteinander verbundenen Zwischenräumen, von 60 % bis 100 % aufweisen.

Weiterhin können miteinander verbundene Zwischenräume der osteokonduktiven Anordnung bzw. Gerüststruktur entlang einer geraden Strecke eine innere Ausdehnung von 200 µm bis 600 µm aufweisen.

Die Stützkörper der ersten Gruppe und/oder die Stützkörper der zweiten Gruppe, insbesondere die Stützkörper der ersten Gruppe und die Stützkörper der zweiten Gruppe, können weiterhin wenigstens eine Abmessung oder wenigstens eine Dimension in einem Größenbereich von 0,5 mm bis 5 mm, insbesondere 1 mm bis 5 mm, bevorzugt 2 mm bis 5 mm, aufweisen. Bei der wenigstens einen Abmessung bzw. Dimension kann es sich insbesondere um die Höhe und/oder die Länge und/oder den Durchmesser, insbesondere mittleren Durchmesser (bestimmt nach Distanz zwischen den beiden entferntesten Punkten auf einem Stützkörper), der Stützkörper handeln.

Weiterhin können die Stützkörper der ersten Gruppe jeweils gleich gestaltet sein, d.h. die gleiche Form aufweisen. Bezüglich geeigneter Formen wird auf die noch folgenden Ausführungen Bezug genommen.

Weiterhin können die Stützkörper der zweiten Gruppe jeweils gleich gestaltet sein, d.h. die gleiche Form aufweisen. Bezüglich geeigneter Formen wird ebenfalls auf die noch folgenden Ausführungen Bezug genommen.

Weiterhin können sowohl die Stützkörper der ersten Gruppe als auch die Stützkörper der zweiten Gruppe jeweils gleich gestaltet sein, d.h. die gleiche Form aufweisen. Bezüglich geeigneter Formen wird ebenso auf die noch folgenden Ausführungen Bezug genommen.

Weiterhin können die Stützkörper der ersten Gruppe anders gestaltet sein, d.h. eine andere Form aufweisen, als die Stützkörper der zweiten Gruppe. Bezüglich geeigneter Formen wird ebenfalls auf die noch folgenden Ausführungen Bezug genommen.

Weiterhin können die Stützkörper der ersten Gruppe gleich gestaltet sein, d.h. die gleiche Form aufweisen, und die Stützkörper der zweiten Gruppe unterschiedlich gestaltet sein, d.h. unterschiedliche Formen aufweisen. Bezüglich infrage kommender Formen wird ebenfalls auf die noch folgenden Ausführungen Bezug genommen.

Weiterhin können die Stützkörper der ersten Gruppe unterschiedlich gestaltet sein, d.h. unterschiedliche Formen aufweisen, und die Stützkörper der zweiten Gruppe gleich gestaltet sein, d.h. die gleiche Form aufweisen. Bezüglich infrage kommender Formen wird ebenfalls auf die noch folgenden Ausführungen Bezug genommen.

In weiterer Ausgestaltung der Erfindung sind die Stützkörper der ersten Gruppe und/oder die Stützkörper der zweiten Gruppe, insbesondere die Stützkörper der ersten Gruppe und die Stützkörper der zweiten Gruppe, oligopodenförmig, d.h. in Form von Oligopoden, gestaltet.

Die Oligopoden können konusförmig und insbesondere rotationssymmetrisch gestaltete Beine aufweisen. Die Beine können einen Konuswinkel von 5° bis 25°, insbesondere 7° bis 15°, aufweisen.

Weiterhin können die Oligopoden Beine mit einer Länge von 0,5 mm bis 5 mm, insbesondere 1,5 mm bis 2,5 mm, aufweisen.

Weiterhin können die Oligopoden Beine mit einem Durchmesser, insbesondere mittleren Durchmesser (bestimmt nach Durchmesser am Punkt in der Mitte vom Stützkörperschwerpunkt und maximale Entfernung vom Schwerpunkt), von 0,2 mm bis 3 mm, insbesondere 0,3 mm bis 0,7 mm, aufweisen.

Die Stützkörper können insbesondere tripodenförmig, tetrapodenförmig, pentapodenförmig, hexapodenförmig, heptapodenförmig oder oktapodenförmig gestaltet sein.

Insbesondere können die Stützkörper der ersten Gruppe eine andere Oligopodenform aufweisen als die Stützkörper der zweiten Gruppe. Bezüglich geeigneter oligopodenförmiger Ausgestaltungen wird auf den vorherigen Absatz Bezug genommen.

Vorzugsweise sind die Stützkörper der ersten Gruppe und/oder die Stützkörper der zweiten Gruppe, insbesondere die Stützkörper der ersten Gruppe und die Stützkörper der zweiten Gruppe, tetrapodenförmig gestaltet. Tetrapodenförmige Stützkörper haben den Vorteil, dass sie beispielsweise durch Impaktieren, d.h. Verdichten und/oder Pressen, des Implantats besonders gut in eine osteokonduktive Anordnung bzw. Gerüststruktur überführbar sind.

In weiterer Ausgestaltung der Erfindung sind die Stützkörper der ersten Gruppe und/oder die Stützkörper der zweiten Gruppe, insbesondere die Stützkörper der ersten Gruppe und die Stützkörper der zweiten Gruppe, polyederförmig gestaltet. Beispielsweise können die Stützkörper quader-, würfel-, tetraeder-, prismen-, pyramiden-, pyramidenstumpf- oder spatförmig gestaltet sein.

Insbesondere können die Stützkörper der ersten Gruppe eine andere Polyederform aufweisen als die Stützkörper der zweiten Gruppe. Bezüglich geeigneter Polyederformen wird auf den vorherigen Absatz Bezug genommen.

Vorzugsweise sind die Stützkörper der ersten Gruppe und/oder die Stützkörper der zweiten Gruppe, insbesondere die Stützkörper der ersten Gruppe und die Stützkörper der zweiten Gruppe, tetraederförmig gestaltet. Tetraederförmige Stützkörper haben (ebenfalls) den Vorteil, dass sie sich beispielsweise durch Impkatieren, d.h. Verdichten und/oder Pressen, des Implantats besonders gut in eine osteokonduktive Anordnung bzw. Gerüststruktur überführen lassen.

Weiterhin können die Stützkörper der ersten Gruppe und/oder die Stützkörper der zweiten Gruppe, insbesondere die Stützkörper der ersten Gruppe und die Stützkörper der zweiten Gruppe, nicht-polyederförmig, insbesondere kugel-, kegel-, kegelstumpf-, ring-, toroid- oder kreiszylinderförmig, gestaltet sein.

Insbesondere können die Stützkörper der ersten Gruppe eine andere Nicht-Polyederform aufweisen als die Stützkörper der zweiten Gruppe. Bezüglich geeigneter Nicht-Polyederformen wird auf den vorherigen Absatz Bezug genommen.

Weiterhin können die Stützkörper der ersten Gruppe und/oder die Stützkörper der zweiten Gruppe, insbesondere die Stützkörper der ersten Gruppe und die Stützkörper der zweiten Gruppe, einen polygonen Querschnitt aufweisen. Beispielsweise können die Stützkörper einen dreieckförmigen, quadratförmigen, rechteckförmigen, fünfeckförmigen, sechseckförmigen, siebeneckförmigen, achteckförmigen, neuneckförmigen, zehneckförmigen oder sternenförmigen Querschnitt aufweisen.

Insbesondere können die Stützkörper der ersten Gruppe einen anderen polygonen Querschnitt aufweisen als die Stützkörper der zweiten Gruppe. Bezüglich geeigneter polygoner Querschnitte wird auf den vorherigen Absatz verwiesen.

Weiterhin können die Stützkörper der ersten Gruppe und/oder die Stützkörper der zweiten Gruppe, insbesondere die Stützkörper der ersten Gruppe und die Stützkörper der zweiten Gruppe, einen eckenlosen, d.h. runden, Querschnitt aufweisen. Beispielsweise können die Stützkörper einen ovalförmigen, insbesondere kreisförmigen oder elliptischen, Querschnitt aufweisen.

Insbesondere können die Stützkörper der ersten Gruppe einen anderen eckenlosen Querschnitt aufweisen als die Stützkörper der zweiten Gruppe. Bezüglich geeigneter eckenloser Querschnitte wird auf den vorherigen Absatz verwiesen.

Weiterhin können die Stützkörper der ersten Gruppe und/oder die Stützkörper der zweiten Gruppe, insbesondere die Stützkörper der ersten Gruppe und die Stützkörper der zweiten Gruppe, mit Vertiefungen und/oder Durchbrüchen versehen sein. Dadurch lässt sich mit besonderem Vorteil ein Einwachsen von Knochengewebe in eine von den Stützkörpern der ersten und zweiten Gruppe gebildete osteokonduktive Anordnung bzw. Gerüststruktur zusätzlich verbessern.

In weiterer Ausgestaltung der Erfindung weisen die Stützkörper der ersten Gruppe und/oder die Stützkörper der zweiten Gruppe, insbesondere die Stützkörper der ersten Gruppe und die Stützkörper der zweiten Gruppe, wenigstens ein Verbindungselement, d.h. ein Verbindungselement oder eine Vielzahl von Verbindungselementen, zum Verbinden des Implantats mit einem Knochen auf. Dadurch kann mit besonderem Vorteil das Implantat-Knochen-Interface zusätzlich verbessert werden, indem die Verbindungselemente die Reibung des Implantats gegenüber Knochen erhöhen und mithin die Verbindung zwischen dem Implantat und dem zu behandelnden Knochendefekt verbessern.

Das wenigstens eine Verbindungselement ist vorzugsweise länglich gestaltet und weist insbesondere ein schmal zulaufendes oder zugespitztes Ende auf. Bevorzugt ist das wenigstens eine Verbindungselement als wenigstens ein Spike gestaltet. Mit anderen Worten können die Stützkörper der ersten Gruppe und/oder die Stützkörper der zweiten Gruppe jeweils ein als Spike gestaltetes Verbindungselement oder eine Vielzahl von jeweils als Spike gestalteten Verbindungselementen aufweisen, wobei das Verbindungselement bzw. die Verbindungselemente zum Verbinden des Implantats mit einem Knochen ausgebildet ist bzw. sind. Unter dem Ausdruck "Spike" soll im Sinne der vorliegenden Erfindung ein längliches und an einem Ende schmal oder spitz zulaufendes Strukturelement verstanden werden.

Vorzugsweise ist das wenigstens eine Verbindungselement an wenigstens einer Ecke von oligopodenförmigen, vorzugsweise tetrapodenförmigen, und/oder polyederförmigen, vorzugsweise tetraederförmigen, Stützkörpern der ersten Gruppe und/oder an wenigstens einer Ecke von oligopodenförmigen, vorzugsweise tetrapodenförmigen, und/oder polyederförmigen, vorzugsweise tetraederförmigen, Stützkörpern der zweiten Gruppe ausgebildet. Insbesondere kann/können das Verbindungselement/die Verbindungselemente an einer Ecke/an Ecken von oligopodenförmigen, vorzugsweise tetrapodenförmigen, und/oder polyederförmigen, vorzugsweise tetraederförmigen, Stützkörpern der ersten Gruppe und/oder an einer Ecke/an Ecken von oligopodenförmigen, vorzugsweise tetrapodenförmigen, und/oder polyederförmigen, vorzugsweise tetraederförmigen, Stützkörpern der zweiten Gruppe ausgebildet sein.

Im Falle von tetraederförmigen Strukturelementen ist das wenigstens eine Verbindungelement vorzugsweise an wenigstens einer Ecke, insbesondere an einer Ecke, an zwei Ecken, an drei Ecken oder an allen (vier) Ecken, der tetraederförmigen Strukturelemente ausgebildet.

Das wenigstens eine Verbindungselement kann eine Länge von 1 mm bis 8 mm, insbesondere 2 mm bis 6 mm, vorzugsweise 3 mm bis 4 mm, aufweisen.

Weiterhin kann das wenigstens eine Verbindungselement einen Durchmesser von 0,01 mm bis 2 mm, insbesondere 0,1 mm bis 1 mm, vorzugsweise 0,2 mm bis 0,7 mm, aufweisen.

Weiterhin kann das wenigstens eine Verbindungselement wenigstens abschnittsweise, insbesondere nur abschnittsweise oder durchgehend, einen eckenlosen, d.h. runden, Querschnitt aufweisen. Beispielsweise kann das wenigstens eine Verbindungselement einen ovalförmigen, insbesondere kreisförmigen oder elliptischen, Querschnitt aufweisen.

Insbesondere kann das wenigstens eine Verbindungselement wenigstens abschnittsweise, insbesondere nur abschnittsweise oder durchgehend, zylindrisch, bevorzugt kreiszylindrisch, gestaltet sein.

Weiterhin kann das wenigstens eine Verbindungselement wenigstens abschnittsweise, insbesondere nur abschnittsweise oder durchgehend, einen polygonen Querschnitt aufweisen. Bei dem polygonen Querschnitt kann es sich beispielsweise um einen dreieckförmigen, quadratförmigen, rechteckförmigen, fünfeckförmigen, sechseckförmigen, siebeneckförmigen, achteckförmigen, neuneckförmigen, zehneckförmigen oder sternenförmigen Querschnitt handeln.

Weiterhin kann das wenigstens eine Verbindungselement wenigstens abschnittsweise, insbesondere nur abschnittsweise oder durchgehend, prismatisch gestaltet sein. Beispielsweise kann das wenigstens eine Verbindungselement wenigstens abschnittsweise, insbesondere nur abschnittsweise oder durchgehend, als gerades Prisma oder schiefes Prisma gestaltet sein.

Weiterhin kann das wenigstens eine Verbindungselement wenigstens abschnittsweise, insbesondere nur abschnittsweise oder durchgehend, quaderförmig gestaltet sein.

Weiterhin kann das wenigstens eine Verbindungselement wenigstens abschnittsweise, insbesondere nur abschnittsweise oder durchgehend, stäbchen- oder stegförmig gestaltet sein.

Weiterhin können die Stützkörper der ersten Gruppe und/oder die Stützkörper der zweiten Gruppe, insbesondere die Stützkörper der ersten Gruppe und die Stützkörper der zweiten Gruppe, längliche Strukturelemente aufweisen oder aus derartigen Strukturelementen zusammengesetzt sein.

Unter dem Ausdruck "längliche Strukturelemente" sollen im Sinne der vorliegenden Erfindung Strukturelemente mit einem Länge-Breite-Verhältnis oder Länge-Durchmesser-Verhältnis > (gesprochen: größer) 1 verstanden werden.

Die länglichen Strukturelemente können eine Länge von 0,4 mm bis 5 mm, insbesondere 1 mm bis 5 mm, bevorzugt 2 mm bis 5 mm, aufweisen.

Weiterhin können die länglichen Strukturelemente einen Durchmesser von 0,4 mm bis 5 mm, insbesondere 1 mm bis 5 mm, bevorzugt 2 mm bis 5 mm, aufweisen. Unter dem Ausdruck "Durchmesser" soll an dieser Stelle der größtmögliche Abstand verstanden werden, welchen zwei Punkte zueinander entlang einer Umfangslinie der länglichen Strukturelemente einnehmen können.

Weiterhin können die länglichen Strukturelemente wenigstens abschnittsweise, insbesondere nur abschnittsweise oder durchgehend, einen eckenlosen, d.h. runden, Querschnitt aufweisen. Beispielsweise können die länglichen Strukturelemente einen ovalförmigen, insbesondere kreisförmigen oder elliptischen, Querschnitt aufweisen.

Insbesondere können die länglichen Strukturelemente wenigstens abschnittsweise, insbesondere nur abschnittsweise oder durchgehend, zylindrisch, bevorzugt kreiszylindrisch, gestaltet sein.

Weiterhin können die länglichen Strukturelemente wenigstens abschnittsweise, insbesondere nur abschnittsweise oder durchgehend, einen polygonen Querschnitt aufweisen. Bei dem polygonen Querschnitt kann es sich beispielsweise um einen dreieckförmigen, quadratförmigen, rechteckförmigen, fünfeckförmigen, sechseckförmigen, siebeneckförmigen, achteckförmigen, neuneckförmigen, zehneckförmigen oder sternenförmigen Querschnitt handeln.

Weiterhin können die länglichen Strukturelemente wenigstens abschnittsweise, insbesondere nur abschnittsweise oder durchgehend, prismatisch gestaltet sein. Beispielsweise können die länglichen Strukturelemente wenigstens abschnittsweise, insbesondere nur abschnittsweise oder durchgehend, als gerades Prisma oder schiefes Prisma gestaltet sein.

Weiterhin können die länglichen Strukturelemente wenigstens abschnittsweise, insbesondere nur abschnittsweise oder durchgehend, quaderförmig gestaltet sein.

Weiterhin können die länglichen Strukturelemente wenigstens abschnittsweise, insbesondere nur abschnittsweise oder durchgehend, stäbchen- oder stegförmig gestaltet sein.

Weiterhin kann das Implantat in sterilisierbarer oder sterilisierter Form vorliegen. Beispielsweise kann das Implantat mittels Gammastrahlen sterilisiert sein.

Weiterhin offenbart ist ein Verfahren zur Herstellung des erfindungsgemäßen Implantats.

Das Verfahren zeichnet sich besonders dadurch aus, dass eine entsprechende Bindemittelmatrix sowie entsprechende Stützkörper unter Erhalt des Implantats gemischt werden. Insbesondere können die Stützkörper der ersten Gruppe sowie die Stützkörper der zweiten Gruppe zu der Bindemittelmatrix hinzugegeben werden.

### KURZBESCHREIBUNG DER FIGUREN

Die Figuren zeigen schematisch Folgendes:
- Fig. 1:: eine Schnittansicht einer Ausführungsform eines erfindungsgemäßen Implantats,
- Fig. 2A:: eine mögliche Ausgestaltung der Stützkörper der ersten und/oder zweiten Gruppe,
- Fig. 2B:: eine weitere mögliche Ausgestaltung der Stützkörper der ersten und/oder zweiten Gruppe,
- Fig. 2C:: eine weitere mögliche Ausgestaltung der Stützkörper der ersten und/oder zweiten Gruppe und
- Fig. 2D:: eine weitere mögliche Ausgestaltung der Stützkörper der ersten und/oder zweiten Gruppe.

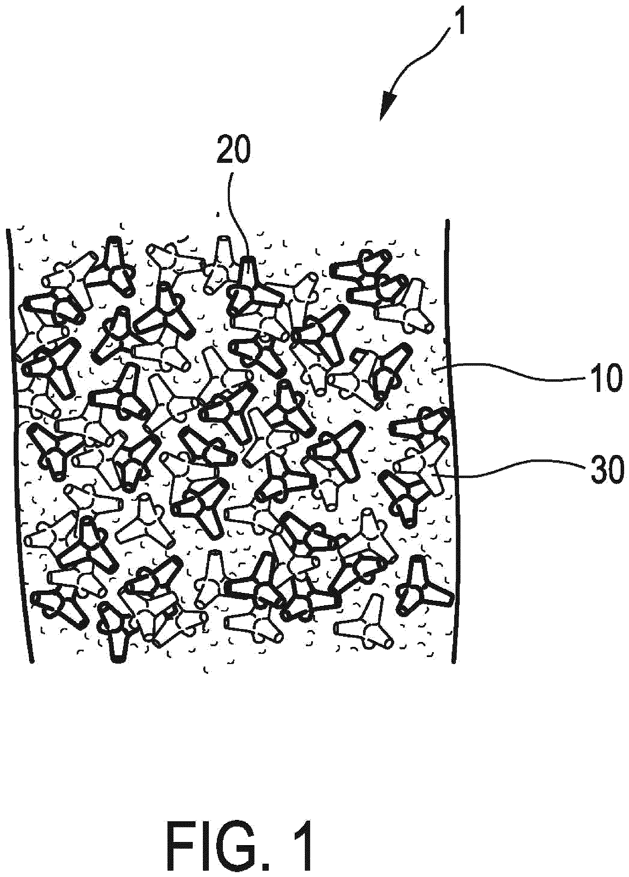

Fig. 1 zeigt schematisch eine Schnittansicht einer Ausführungsform eines erfindungsgemäßen Implantats 1.

Das Implantat 1 weist eine Bindemittelmatrix 10 sowie eine erste Gruppe von Stützkörpern 20 und eine zweite Gruppe von Stützkörpern 30 auf. Die Stützkörper 20;30 können, wie dargestellt, bevorzugt tetrapodenförmig gestaltet sein.

Die Stützkörper 20 und 30 liegen innerhalb der Bindemittelmatrix 10 verteilt vor.

Sowohl die Stützkörper 20 der ersten Gruppe als auch die Stützkörper 30 der zweiten Gruppe sind in vivo abbaubar und/oder in vivo resorbierbar, wobei die Stützkörper 20 der ersten Gruppe schneller in vivo abbaubar und/oder schneller in vivo resorbierbar sind als die Stützkörper 30 der zweiten Gruppe.

Bevorzugt bilden die Stützkörper 20 der ersten Gruppe sowie die Stützkörper 30 der zweiten Gruppe, insbesondere in einem impaktierten Zustand des Implantats, durch gegenseitige Berührung eine osteokonduktive Anordnung oder osteokonduktive Gerüststruktur, d.h. eine Anordnung bzw. Gerüststruktur mit Zwischenräumen, welche ein Einwachsen von Knochengewebe erlauben.

Die Bindemittelmatrix 10 kann mittels Wasser und/oder einer wässrigen Flüssigkeit, wie beispielsweise einer medizinisch verträglichen, wässrigen Spülflüssigkeit, entfernbar sein. Insbesondere kann es sich bei der Bindemittelmatrix 10 um eine wasserlösliche Bindemittelmatrix handeln.

Alternativ kann die Bindemittelmatrix 10 schneller in vivo abbaubar und/oder schneller in vivo resorbierbar sein als die Stützkörper 20 der ersten Gruppe sowie die Stützkörper 30 der zweiten Gruppe.

Dadurch lassen sich mit besonderem Vorteil die drei für eine erfolgreiche Revisionsoperation, insbesondere Hüftrevisionsoperation, maßgeblichen Ziele, nämlich die Wiederherstellung des Gelenkzentrums, die Erreichung einer ausreichenden Primär- sowie Sekundärstabilität des Implantats sowie die biologische Rekonstruktion des zu behandelnden Knochendefekts erreichen.

Dadurch, dass die Bindemittelmatrix 10 durch Wasser und/oder eine wässrigen Flüssigkeit entfernbar und/oder schneller in vivo abbaubar und/oder schneller in vivo resorbierbar ist als die Stützkörper 20 der ersten Gruppe und die Stützkörper 30 der zweiten Gruppe, kann mit besonderem Vorteil während einer anfänglichen Behandlungs- oder Heilungsphase ein zum Einwachsen von Knochengewebe erforderlicher Platz oder Raum geschaffen werden. Die Primär- sowie Sekundärstabilität des Implantats wird während dieser Phase sowohl durch die Stützkörper 20 der ersten Gruppe als auch durch die Stützkörper 30 der zweiten Gruppe gewährleistet.

Während einer zweiten Heilungsphase werden die Stützkörper 20 der ersten Gruppe aufgrund ihrer im Verhältnis zu den Stützkörpern 30 der zweiten Gruppe schnelleren In-vivo-Abbau- und/oder Resorptionsgeschwindigkeit zu Knochen ab- und/oder umgebaut. Durch den Abbau und/oder die Resorption der Stützkörper 20 wird weiterer Platz bzw. Raum zum Einwachsen von Knochengewebe geschaffen, so dass die biologische Rekonstruktion des Knochendefekts kontinuierlich fortschreiten kann. Während dieser Phase wird die mechanische Stabilität (Sekundärstabilität) von den Stützkörpern 30 der zweiten Gruppe übernommen.

Während einer dritten Heilungshase werden schließlich auch die Stützkörper 30 der zweiten Gruppe zu Knochen ab- und/oder umgebaut. Aufgrund des Abbaus und/oder der Resorption der Stützkörper 30 wird zusätzlicher Platz bzw. Raum zum Einwachsen von Knochengewebe geschaffen, so dass die biologische Rekonstruktion des Knochendefekts auch während dieser Phase kontinuierlich fortschreiten kann. Nach vollständigem Abbau und/oder vollständiger Resorption der Stützkörper 30 ist die dritte Phase und mithin die biologische Rekonstruktion des Knochendefekts abgeschlossen, wobei die mechanische Stabilität während des gesamten Zeitraums über gewährleistet war.

Die Bindemittelmatrix kann hierzu Polyethylenglykol (PEG), insbesondere PEG 400 und/oder PEG 1.500, und optional Glycerin aufweisen oder aus Polyethylenglykol (PEG), insbesondere PEG 400 und/oder PEG 1.500, und optional Glycerin bestehen. Beispielsweise kann die Bindemittelmatrix PEG 400 und PEG 1.500 in einem Verhältnis von 1,5 : 1 zu 4 : 1 aufweisen.

Weiterhin kann die Bindemittelmatrix PEG 400, PEG 1.500 sowie Glycerin in einem Verhältnis (PEG 400 : PEG 1.500 : Glycerin) von 3 : 2: 1 oder 4 : 1 : 1 aufweisen.

Die Stützkörper 20 der zweiten Gruppe weisen bevorzugt β-Tricalciumphosphat auf oder bestehen aus β-Tricalciumphosphat.

Die Stützkörper 30 der zweiten Gruppe weisen bevorzugt biphasisches Calciumphosphat auf oder bestehen aus biphasischem Calciumphosphat.

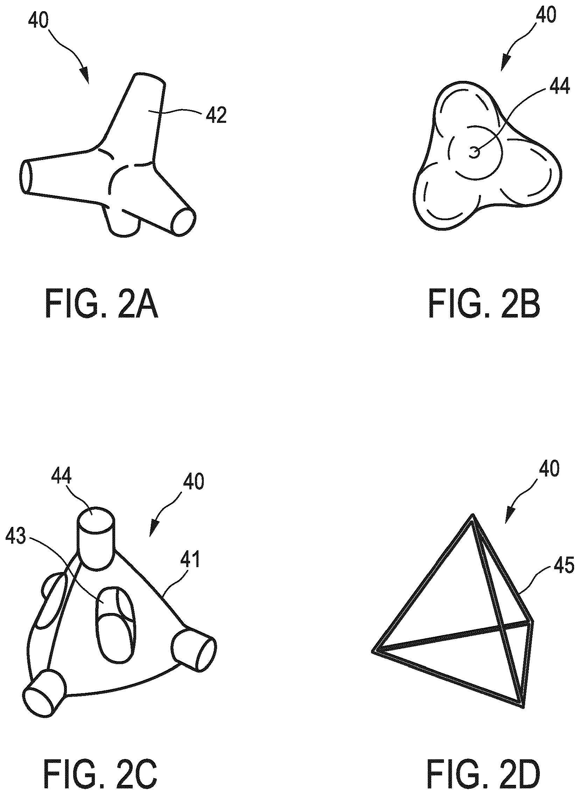

Fig. 2A zeigt schematisch eine mögliche Ausgestaltung oder Form, welche die Stützkörper der ersten Gruppe und/oder die Stützkörper der zweiten Gruppe, vorzugsweise die Stützkörper der ersten Gruppe und die Stützkörper der zweiten Gruppe, aufweisen können. Der dargestellte Tetraeder 40 besitzt eine Tetrapodenform. Dadurch können die Stützkörper des erfindungsgemäßen Implantats, insbesondere durch Verdichten oder Pressen des Implantats, innerhalb der Bindematrix in eine osteokonduktive Anordnung oder Gerüststruktur, d.h. in eine Anordnung bzw. Gerüststruktur mit Zwischenräumen, in welche ein Einwachsen von Knochengewebe stattfinden kann, überführt werden. Dadurch lässt sich die biologische Rekonstruktion des zu behandelnden Knochendefekts mit besonderem Vorteil beschleunigen.

Der tetrapodenförmige Stützkörper 40 kann hierzu konisch gestaltete Tetrapodenbeine 42 aufweisen.

Fig. 2B zeigt schematisch eine weitere mögliche Ausgestaltung oder Form, welche die Stützkörper der ersten Gruppe und/oder die Stützkörper der zweiten Gruppe, vorzugsweise die Stützkörper der ersten Gruppe und die Stützkörper der zweiten Gruppe, des Implantats aufweisen können. Der dargestellte Stützkörper 40 weist ebenfalls eine Tetrapodenform auf. Diese ist jedoch im Verhältnis zur Tetrapodenform des in Fig. 2A dargestellten Stützkörpers flacher und insbesondere gedrungener gestaltet. Obendrein weist die Tetrapodenform des in Fig. 2B dargestellten Stützkörpers 40 an einer Ecke der Tetrapodenform ein Verbindungselement 44 auf. Das Verbindungselement 44 kann, wie dargestellt, wenigstens abschnittsweise kreiszylindrisch gestaltet sein. Das Verbindungselement 44 ermöglicht mit besonderem Vorteil eine Verbesserung des Implantat-Knochen-Interface, indem das Verbindungselement 44 die Reibung des Implantats gegenüber Knochen erhöht und dadurch die Verbindung zwischen dem Implantat und dem zu behandelnden Knochendefekt verbessert.

Fig. 2C zeigt eine weitere mögliche Ausgestaltung oder Form, welche die Stützkörper der ersten Gruppe und/oder die Stützkörper der zweiten Gruppe, bevorzugt die Stützkörper der ersten Gruppe und die Stützkörper der zweiten Gruppe, aufweisen können. Der dargestellte Stützkörper 40 besitzt eine Tetraedergrundstruktur 41, welche Durchbrüche 43 sowie an jeder Tetraederecke ein wenigstens abschnittsweise kreiszylindrisch gestaltetes Verbindungselement 44 aufweist. Während die Durchbrüche 43 ein Einwachsen von Knochengewebe fördern, bewirken die Verbindungselemente 44, wie oben bereits erwähnt, eine zusätzliche Verbesserung des Implantat-Knochen-Interface.

Fig. 2D zeigt eine weitere mögliche Ausgestaltung oder Form, welche die Stützkörper der ersten Gruppe und/oder die Stützkörper der zweiten Gruppe, bevorzugt die Stützkörper der ersten Gruppe und die Stützkörper der zweiten Gruppe, aufweisen können.

Der dargestellte Stützkörper 40 weist eine Tetraederform auf, wobei die Tetraederform aus länglichen Strukturelementen 45 zusammengesetzt ist. Aufgrund des Platzes oder Raums zwischen den Strukturelementen 45 lassen sich Stützkörper, welche die in Fig. 2D gezeigte Anordnung oder Form besitzen, beim Impaktieren des Implantats besonders einfach in eine osteokonduktive Anordnung bzw. Gerüststruktur überführen.

### AUSFÜHRUNGSBEISPIEL

Mittels Pulverspritzguss wurden sowohl tetrapodenförmige Stützkörper aus beta-Tricalciumphosphat als auch tetrapodenförmige Stützkörper aus biphasischem Calciumphosphat (50 Gew.-% beta-Tricalciumphosphat und 50 Gew.-% Hydroxylapatit) hergestellt. Die Herstellung erfolgte in einem Werkzeug mit 25 Kavitäten, wodurch mit jedem Schuss 25 tetrapodenförmige Stützkörper gefertigt werden konnten. Die tetrapodenförmigen Stützkörper hatten jeweils eine Gesamtausdehnung von 4 mm.

Die Bindemittelmatrix wurde aus 4 Anteilen PEG 400, 1 Anteil PEG 1500 und 1 Anteil Glycerin mittels Hitze und Rühren hergestellt.

Anschließend wurden die Stützkörper mit der Bindemittelmatrix in einem Gewichtsverhältnis 10 : 4 gemischt. Die fertige Mischung wurde im Reinraum verpackt und mit Gammastrahlen sterilisiert.

Die fertige Mischung bestand aus Tetrapoden aus beta-Tricalciumphosphat, Tetrapoden aus biphasischem Calciumphosphat und aus der Bindemittelmatrix.

Nach Öffnung der Verpackung kann die fertige Mischung bzw. das Implantat in einen Knochendefekt angebracht werden.

## Patentansprüche

1. Implantat, vorzugsweise zum Behandeln eines Knochendefekts, aufweisend
- eine Bindemittelmatrix,
- eine erste Gruppe von Stützkörpern und
- eine zweite Gruppe von Stützkörpern,
wobei die Stützkörper von der Bindemittelmatrix wenigstens teilweise umgeben sind,
**dadurch gekennzeichnet, dass**
- die Bindemittematrix mittels Wasser und/oder einer wässrigen Flüssigkeit entfernbar ist und die Stützkörper der ersten Gruppe schneller in vivo abbaubar und/oder schneller in vivo resorbierbar sind als die Stützkörper der zweiten Gruppe
und/oder
- die Bindemittematrix schneller in vivo abbaubar und/oder schneller in vivo resorbierbar ist als die Stützkörper der ersten und zweiten Gruppe und die Stützkörper der ersten Gruppe schneller in vivo abbaubar und/oder schneller in vivo resorbierbar sind als die Stützkörper der zweiten Gruppe.

2. Implantat nach Anspruch 1, **dadurch gekennzeichnet, dass** die Bindemittelmatrix plastisch verformbar, insbesondere knetbar, ist.

3. Implantat nach Anspruch 1 oder 2, **dadurch gekennzeichnet, dass** es sich bei der Bindemittelmatrix um eine wasserlösliche Bindemittelmatrix handelt.

4. Implantat nach einem der vorhergehenden Ansprüche, **dadurch gekennzeichnet, dass** die Bindemittelmatrix ein anderes Material aufweist oder aus einem anderen Material besteht als die Stützkörper der ersten und zweiten Gruppe.

5. Implantat nach einem der vorhergehenden Ansprüche, **dadurch gekennzeichnet, dass** die Bindemittelmatrix Polyethylenglykol aufweist oder aus Polyethylenglykol besteht, wobei das Polyethylenglykol vorzugsweise ausgewählt ist aus der Gruppe bestehend aus PEG 400, PEG 1500 und Mischungen davon.

6. Implantat nach Anspruch 5, **dadurch gekennzeichnet, dass** die Bindemittelmatrix eine Mischung von PEG 400 und PEG 1500 aufweist.

7. Implantat nach Anspruch 6 **dadurch gekennzeichnet, dass** die Bindemittelmatrix ein Verhältnis von PEG 400 zu PEG 1500 von 1,5 : 1 bis 4 : 1 aufweist.

8. Implantat nach einem der vorhergehenden Ansprüche, **dadurch gekennzeichnet, dass** die Bindemittelmatrix ferner Glycerin aufweist.

9. Implantat nach einem der vorhergehenden Ansprüche, **dadurch gekennzeichnet, dass** die Bindemittelmatrix ein Verhältnis von PEG 400 zu PEG 1500 zu Glycerin von 3 : 2 : 1 oder 4 : 1 : 1 aufweist.

10. Implantat nach einem der vorhergehenden Ansprüche, **dadurch gekennzeichnet, dass** die Bindemittelmatrix einen Anteil von 10 Gew.-% bis 60 Gew.-% aufweist, bezogen auf das Gesamtgewicht des Implantats, und/oder die Stützkörper insgesamt einen Anteil von 40 Gew.-% bis 90 Gew.-% aufweisen, bezogen auf das Gesamtgewicht des Implantats.

11. Implantat nach einem der vorhergehenden Ansprüche, **dadurch gekennzeichnet, dass** die Stützkörper der ersten Gruppe ein anderes Material aufweisen oder aus einem anderen Material bestehen als die Stützkörper der zweiten Gruppe.

12. Implantat nach einem der vorhergehenden Ansprüche, **dadurch gekennzeichnet, dass** die Stützkörper der ersten Gruppe beta-Tricalciumphosphat aufweisen oder aus beta-Tricalciumphosphat bestehen.

13. Implantat nach einem der vorhergehenden Ansprüche, **dadurch gekennzeichnet, dass** die Stützkörper der zweiten Gruppe Hydroxylapatit aufweisen oder aus Hydroxylapatit bestehen, oder die Stützkörper der zweiten Gruppe eine Mischung von beta-Tricalciumphosphat und Hydroxylapatit aufweisen oder aus einer solchen Mischung bestehen.

14. Implantat nach einem der vorhergehenden Ansprüche, **dadurch gekennzeichnet, dass** die Stützkörper oligopodenförmig, vorzugsweise tetrapodenförmig, und/oder polyederförmig, vorzugsweise tetraederförmig, gestaltet sind.

15. Implantat nach einem der vorhergehenden Ansprüche, **dadurch gekennzeichnet, dass** die Stützkörper der ersten Gruppe und/oder die Stützkörper der zweiten Gruppe wenigstens ein Verbindungselement, insbesondere eine Vielzahl von Verbindungselementen, zum Verbinden des Implantats mit einem Knochen aufweisen, wobei das wenigstens eine Verbindungselement, insbesondere die Vielzahl von Verbindungselementen, an einer Ecke, insbesondere an Ecken, von oligopodenförmigen und/oder polyederförmigen Stützkörpern der ersten Gruppe und/oder an einer Ecke, insbesondere an Ecken, von oligopodenförmigen und/oder polyederförmigen Stützkörpern der zweiten Gruppe ausgebildet ist.

## Claims

1. Implant, preferably for treatment of a bone defect, comprising
- a binder matrix,
- a first group of support bodies and
- a second group of support bodies,
wherein the support bodies are at least partially surrounded by the binder matrix,
**characterized in that**
- the binder matrix is removable by means of water and/or an aqueous liquid and the support bodies of the first group are more rapidly in vivo degradable and/or more rapidly in vivo resorbable than the support bodies of the second group
and/or
- the binder matrix is more rapidly in vivo degradable and/or more rapidly in vivo resorbable than the support bodies of the first and second group and the support bodies of the first group are more rapidly in vivo degradable and/or more rapidly in vivo resorbable than the support bodies of the second group.

2. Implant according to Claim 1, **characterized in that** the binder matrix is plastically deformable, especially kneadable.

3. Implant according to Claim 1 or 2, **characterized in that** the binder matrix is a water-soluble binder matrix.

4. Implant according to any of the preceding claims, **characterized in that** the binder matrix comprises or consists of a different material than the support bodies of the first and second group.

5. Implant according to any of the preceding claims, **characterized in that** the binder matrix comprises or consists of polyethylene glycol, wherein the polyethylene glycol is preferably selected from the group consisting of PEG 400, PEG 1500 and mixtures thereof.

6. Implant according to Claim 5, **characterized in that** the binder matrix comprises a mixture of PEG 400 and PEG 1500.

7. Implant according to Claim 6, **characterized in that** the binder matrix has a ratio of PEG 400 to PEG 1500 of from 1.5:1 to 4:1.

8. Implant according to any of the preceding claims, **characterized in that** the binder matrix further comprises glycerol.

9. Implant according to any of the preceding claims, **characterized in that** the binder matrix has a ratio of PEG 400 to PEG 1500 to glycerol of 3:2.1 or 4:1:1.

10. Implant according to any of the preceding claims, **characterized in that** the proportion of binder matrix is from 10% by weight to 60% by weight, based on the total weight of the implant, and/or the proportion of support bodies as a whole is from 40% by weight to 90% by weight, based on the total weight of the implant.

11. Implant according to any of the preceding claims, **characterized in that** the support bodies of the first group comprise or consist of a different material than the support bodies of the second group.

12. Implant according to any of the preceding claims, **characterized in that** the support bodies of the first group comprise or consist of beta-tricalcium phosphate.

13. Implant according to any of the preceding claims, **characterized in that** the support bodies of the second group comprise or consist of hydroxyapatite or the support bodies of the second group comprise or consist of a mixture of beta-tricalcium phosphate and hydroxyapatite.

14. Implant according to any of the preceding claims, **characterized in that** the support bodies are oligopodal, preferably tetrapodal, and/or polyhedral, preferably tetrahedral.

15. Implant according to any of the preceding claims, **characterized in that** the support bodies of the first group and/or the support bodies of the second group comprise at least one connection element, especially a multiplicity of connection elements, for connection of the implant to a bone, wherein the at least one connection element, especially the multiplicity of connection elements, is formed at a corner, especially at corners, of oligopodal and/or polyhedral support bodies of the first group and/or at a corner, especially at corners, of oligopodal and/or polyhedral support bodies of the second group.

## Revendications

1. Implant, de préférence pour le traitement d'un défaut osseux, présentant
- une matrice d'agent liant,
- un premier groupe de corps support et
- un deuxième groupe de corps support,
les corps support étant au moins partiellement entourés par la matrice d'agent liant, **caractérisé en ce que**
- la matrice d'agent liant peut être éliminée au moyen d'eau et/ou d'un liquide aqueux et les corps support du premier groupe sont plus rapidement dégradables in vivo et/ou plus rapidement résorbables in vivo que les corps support du deuxième groupe
et/ou
- la matrice d'agent liant est plus rapidement dégradable in vivo et/ou plus rapidement résorbable in vivo que les corps support du premier et du deuxième groupe et les corps support du premier groupe sont plus rapidement dégradables in vivo et/ou plus rapidement résorbables in vivo que les corps support du deuxième groupe.

2. Implant selon la revendication 1, **caractérisé en ce que** la matrice d'agent liant est plastiquement déformable, en particulier malaxable.

3. Implant selon la revendication 1 ou 2, **caractérisé en ce qu'**il s'agit, pour la matrice d'agent liant, d'une matrice d'agent liant soluble dans l'eau.

4. Implant selon l'une quelconque des revendications précédentes, **caractérisé en ce que** la matrice d'agent liant présente un autre matériau ou est constituée par un autre matériau que celui des corps support du premier et du deuxième groupe.

5. Implant selon l'une quelconque des revendications précédentes, **caractérisé en ce que** la matrice d'agent liant présente du polyéthylèneglycol ou est constituée de polyéthylèneglycol, le polyéthylèneglycol étant de préférence choisi dans le groupe constitué par le PEG 400, le PEG 1500 et leurs mélanges.

6. Implant selon la revendication 5, **caractérisé en ce que** la matrice d'agent liant présente un mélange de PEG 400 et de PEG 1500.

7. Implant selon la revendication 6, **caractérisé en ce que** la matrice d'agent liant présente un rapport de PEG 400 à PEG 1500 de 1,5:1 à 4:1.

8. Implant selon l'une quelconque des revendications précédentes, **caractérisé en ce que** la matrice d'agent liant présente en outre du glycérol.

9. Implant selon l'une quelconque des revendications précédentes, **caractérisé en ce que** la matrice d'agent liant présente un rapport de PEG 400 à PEG 1500 à glycérol de 3:2:1 ou de 4:1:1.

10. Implant selon l'une quelconque des revendications précédentes, **caractérisé en ce que** la matrice d'agent liant représente une proportion de 1% en poids à 60% en poids, par rapport au poids total de l'implant, et/ou les corps support représentent au total une proportion de 40% en poids à 90% en poids, par rapport au poids total de l'implant.

11. Implant selon l'une quelconque des revendications précédentes, **caractérisé en ce que** les corps support du premier groupe présentent un autre matériau ou sont constitués par un autre matériau que celui des corps support du deuxième groupe.

12. Implant selon l'une quelconque des revendications précédentes, **caractérisé en ce que** les corps support du premier groupe présentent du bêta-phosphate tricalcique ou sont constitués de bêta-phosphate tricalcique.

13. Implant selon l'une quelconque des revendications précédentes, **caractérisé en ce que** les corps support du deuxième groupe présentent de l'hydroxylapatite ou sont constitués d'hydroxylapatite ou les corps support du deuxième groupe présentent un mélange de bêta-phosphate tricalcique et d'hydroxylapatite ou sont constitués par un tel mélange.

14. Implant selon l'une quelconque des revendications précédentes, **caractérisé en ce que** les corps support sont réalisés sous forme d'oligopode, de préférence sous forme de tétrapode et/ou sous forme de polyèdre, de préférence sous forme de tétraèdre.

15. Implant selon l'une quelconque des revendications précédentes, **caractérisé en ce que** les corps support du premier groupe et/ou les corps support du deuxième groupe présentent au moins un élément de liaison, en particulier une multitude d'éléments de liaison, pour relier l'implant à un os, ledit au moins un élément de liaison, en particulier la multitude d'éléments de liaison, étant conçu(s) au niveau d'un coin, en particulier au niveau de coins de corps support sous forme d'oligopode et/ou sous forme de polyèdre du premier groupe et/ou au niveau d'un coin, en particulier au niveau de coins de corps support sous forme d'oligopode et/ou sous forme de polyèdre du deuxième groupe.
